# EUROPEAN PATENT SPECIFICATION

(11) **EP 3 156 029 B1**
(45) Date of publication and mention of the grant of the patent: **06.05.2020**
(21) Application number: 16193458.3
(22) Date of filing: 12.10.2016
(51) Int. Cl.: A61H 23/00

(54) **PERCUSSIVE CHEST THERAPY APPARATUS HAVING A NEBULIZER INTERFACE**
BRUSTSCHLAGTHERAPIEVORRICHTUNG MIT EINER NEBULISATORSCHNITTSTELLE
APPAREIL DE THÉRAPIE RESPIRATOIRE À PERCUSSION COMPORTANT UNE INTERFACE NÉBULISEUR

(30) Priority: 13.10.2015 US 201562240704 P
(43) Date of publication of application: 19.04.2017
(73) Proprietor: Hill-Rom Services Pte. Ltd., Singapore 768923 (SG)
(72) Inventor: VENKATARAYA, Suresha, 760136 Singapore (SG)
(74) Representative: Findlay, Alice Rosemary

(56) References cited:
- US-A1- 2013 291 859
- US-A1- 2015 157 532
- US-A1- 2015 273 164

## Description

The present disclosure relates to a high frequency wall oscillation apparatus for applying respiratory therapy to a patient. More particularly, the present disclosure relates to an apparatus to be worn by the patient in order to treat respiratory diseases or conditions.

Often, patients needing treatment of a respiratory disease such as cystic fibrosis use several different types of devices for treatment such as a nebulizer and a high frequency chest wall oscillation (HFCWO) device. Some HFCWO devices include a vest that covers a portion of a patient's thorax and that provides percussive forces to the thorax. Nebulizer treatment involves creating an aerosolized drug stream for delivery to a patient's airways. Both of these devices treat one or more of the patient's respiratory problems. However, a patient typically operates the devices separately in order to receive the benefit of both. Operation of both devices requires manipulating controls of two separate devices. Patent Document US2015/0157532 A1 discloses a wearable apparatus for oscillation chest therapy with a nebulizer.

The present application discloses one or more of the following features alone or in any combination.

According to a first aspect of the present disclosure, a high frequency chest wall oscillation apparatus may include a garment to be worn by a patient, a plurality of mechanical percussors that may be carried by the garment, a controller that may be coupled to the garment and that may signal operation of the mechanical percussors, an electrically operated nebulizer that may removably attach to the controller to receive power therefrom, and a patient interface to introduce nebulized particles from the electrically operated nebulizer into the patient's airway.

In some embodiments, the garment may be a vest that may have a front panel and a rear panel that may be coupled to the front panel by adjustable shoulder straps and/or adjustable side straps. The front panel may be separated into two parts that may be connected by a full frontal closure, and the two parts of the front panel may carry a first set of the mechanical percussors of the plurality of the mechanical percussors and a second set of the mechanical percussors of the plurality of mechanical percussors may be carried by the rear panel.

In some embodiments, there may be a port electrically coupled to the controller and the electrically operated nebulizer that may be coupleable to the port. The port may be a USB port, for example.

In some embodiments, the controller may further include a power supply that may be electrically coupled to the electrically operated nebulizer. The power supply may be a rechargeable power supply.

In some embodiments, the controller may instruct the operation of the electrically operated nebulizer.

In some embodiments, the patient interface to introduce nebulized particles from the electrically operated nebulizer into the patient's airway may be a mouthpiece or a mask or a nasal cannula or combinations of two or three of these.

In some embodiments, the controller may drive the electrically operated nebulizer. The controller may drive the electrically operated nebulizer by electrical voltage. The controller may also drive the electrically operated nebulizer by pneumatic flow.

In some embodiments, the electrically operated nebulizer may be a vibrating mesh nebulizer or a vibrating plate nebulizer.

In some embodiments, the apparatus may further comprise a storage component such as a strap or pocket on the garment for storage of the nebulizer, the patient interface, or both.

In some embodiments, the apparatus may further comprise a switch to change between a plurality of different modes of operation. The modes of operation may include an on state and an off state for the plurality of mechanical percussors, an on state and an off state for the electrically operated nebulizer, and an on state and an off state for the plurality of mechanical percussors and the electrically operated nebulizer.

The invention will now be further described by way of example with reference to the accompanying drawings, in which:
Fig. 1A is a perspective view of a front of a high frequency chest wall oscillation (HFCWO) apparatus including a nebulizer according to the present disclosure;
Fig. 1B is a perspective view similar to Fig. 1 showing a back of the HFCWO apparatus;
Fig. 2 is a perspective view of the HFCWO apparatus being worn by a patient; and
Fig. 3 is a block diagram of the electrical components of the apparatus.

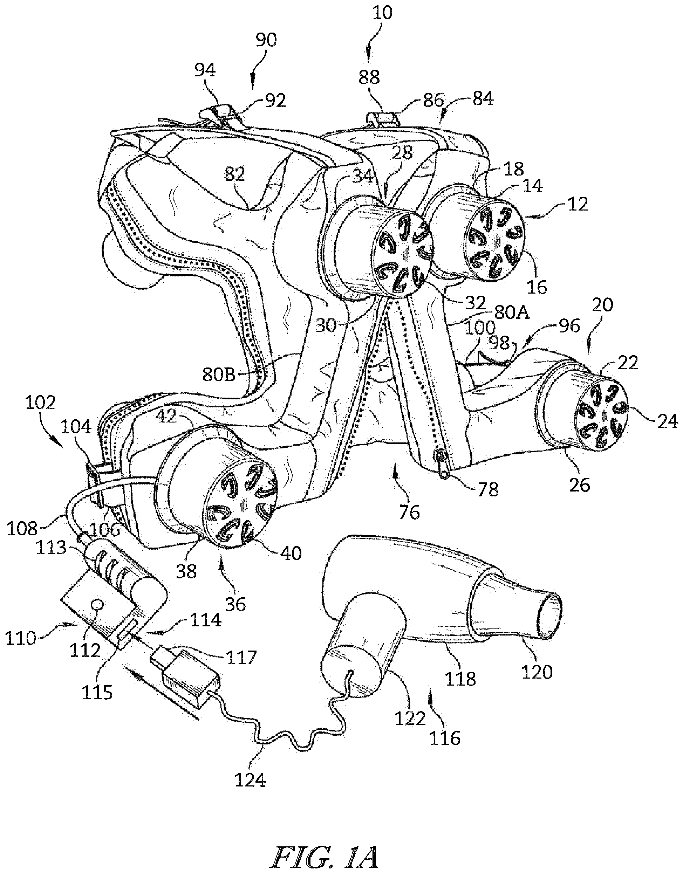

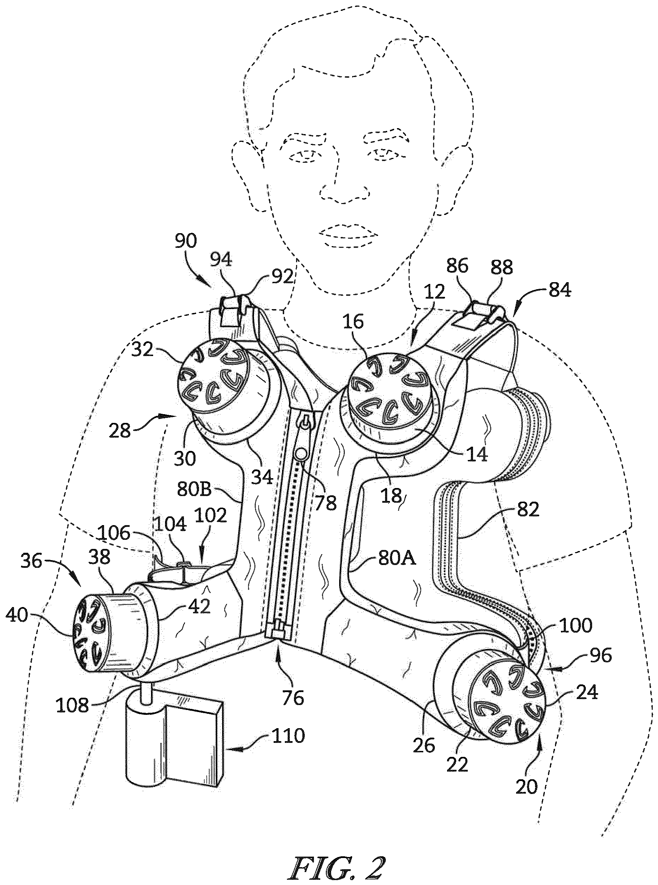

A high frequency chest wall oscillation (HFCWO) apparatus 10 includes a garment 11 which in the illustrative example is configured as a vest as shown in Figs. 1A-1B and Fig. 2. In an example of use, the apparatus 10 assists a patient with cystic fibrosis by dislodging mucous buildup in the airways and by encouraging expectoration of the mucous. The nature of the apparatus 10 allows for patient mobility while receiving treatment. In the illustrative embodiment, the garment 11 includes a front panel 80 divided into a left side 80A and a right side 80B connected by a frontal closure 76 which is illustratively embodied as a zipper 78 as shown in Figs. 1A and 2.

Both sides 80A, 80B of the front panel 80 resemble an L-shaped structure connected by the closure 76 on the long side of the L-shaped structure. The closure 76 allows the garment 11 to be worn and removed by a patient. In the illustrative example, the garment 11 further includes a rear panel 82 which resembles an I-shaped structure. In the illustrative embodiment, the front panel 80 and the rear panel 82 include a padded material to provide a suitable comfort level for the patient wearing the garment 11.

The front panel 80 connects to the rear panel 82 through adjustable shoulder straps 84, 90 of garment 11 and adjustable side straps 96, 102 of garment 11. The adjustable straps 84, 90, 96, 102 each include a respective buckle 86, 92, 98, 104 connected to a corresponding strap 88, 94, 100, 106. The adjustable straps 84, 90, 96, 102 are lengthened and shortened through the respective buckles 86, 92, 98, 104 to provide customization of the garment 11 according to a body size of the patient using apparatus 10. In addition, the adjustable straps 84, 90, 96, 102 enable tightening of the garment 11 onto the patient's thorax to enhance the effectiveness of the treatment.

The apparatus 10 also includes, for example, a number of mechanical percussors 12, 20, 28, 36, 44, 52, 60, 68 as shown in Figs. 1A-1B. The mechanical percussors 12, 20, 28, 36, 44, 52, 60, 68 operate to provide percussive forces onto a patient's thorax at corresponding locations to dislodge mucous buildup in the patient's airways. Additional information regarding the configuration and operation of the mechanical percussors 12, 20, 28, 36, 44, 52, 60, 68 may be found in U.S. Patent Application Publication Nos. 2014/0005579 A1 and 2014/0012167 A1 which are hereby incorporated by reference herein in their entirety to the extent not inconsistent with the present disclosure which shall control as to any inconsistencies.

The mechanical percussors 12, 20, 28, 36, 44, 52, 60, 68 are held by the front panel 80 and the rear panel 82 in order to distribute the percussive forces applied to the patient. The distribution allows the apparatus 10 to target specific areas for targeted treatment. In the illustrative example, the front left side panel 80A holds two mechanical percussors 12, 20, the front right side panel 80B holds two mechanical percussors 28, 36, and the rear panel 82 holds four mechanical percussors 44, 52, 60, 68 as shown in Figs. 1A-1B. Each of the mechanical percussors 12, 20, 28, 36, 44, 52, 60, 68 include respective cylindrical walls 14, 22, 30, 38, 46, 54, 62, 70, respective circular tops 16, 24, 32, 40, 48, 56, 64, 72, and respective flanges 18, 26, 34, 42, 50, 58, 66, 74.

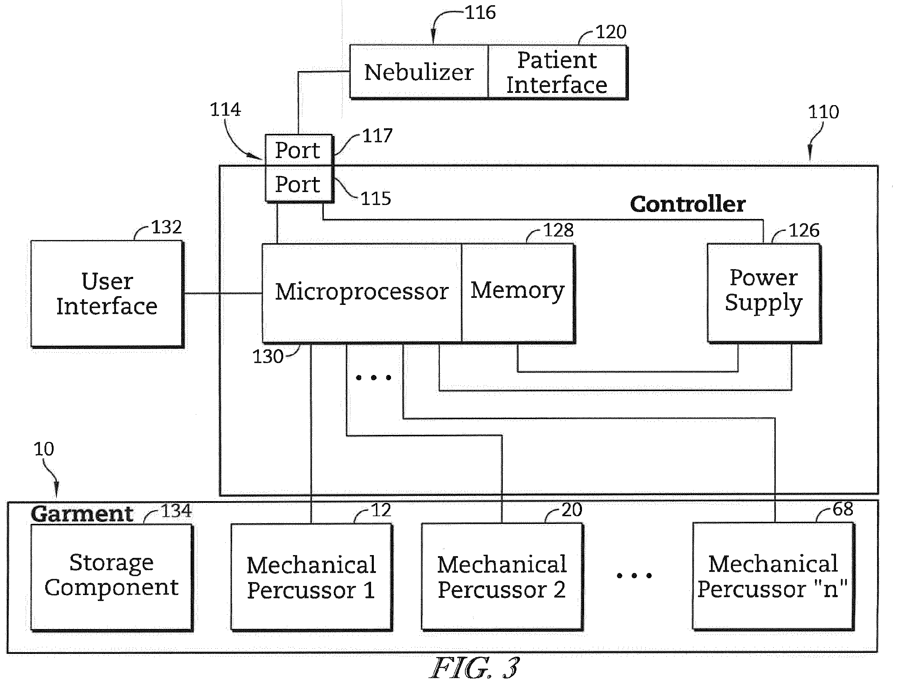

In the illustrative example, apparatus 10 has eight percussors 12, 20, 28, 36, 44, 52, 60, 68. However, it is within the scope of this disclosure for apparatus 10 to have a different number of percussors, such as more or less than eight. Accordingly, Fig. 3 indicates that apparatus 10 has "n" Mechanical Percussors.

The illustrative example of the apparatus 10 further includes a controller 110 with a connecting interface 108. The connecting interface 108 is illustratively embodied as, but is not limited to, a cable 108 as shown in Figs. 1A-1B and Fig. 2. However, the connecting interface 108 between the apparatus 10 and the controller 110 can include alternative connections such as couplers or ports that directly connect to each other without any flexible cable therebetween.

Conductors from cable 108 are routed through garment 11 to each of the mechanical percussors 12, 20, 28, 36, 44, 52, 60, 68 to electrically couple the controller 110 to each of the mechanical percussors 12, 20, 28, 36, 44, 52, 60, 68. The wires are routed through or alongside straps 84, 90, 96, 102 of the garment 11 in some embodiments. In other embodiments, a second cable, like cable 108 extends from controller 108 to rear panel 82. In some embodiments, cable 108 has conductors electrically coupled to percussors 28, 36 of panel 80B and a third cable 108, like cable 108, extends from controller 110 to panel 80A for electrically coupling to percussors 12, 20 of panel 80A. Regardless of the number of cables and the manner of routing of wires through garment 11, suffice it to say that controller 110 commands the operation of each of percussors 12, 20, 28, 36, 44, 52, 60, 68 of the apparatus 10.

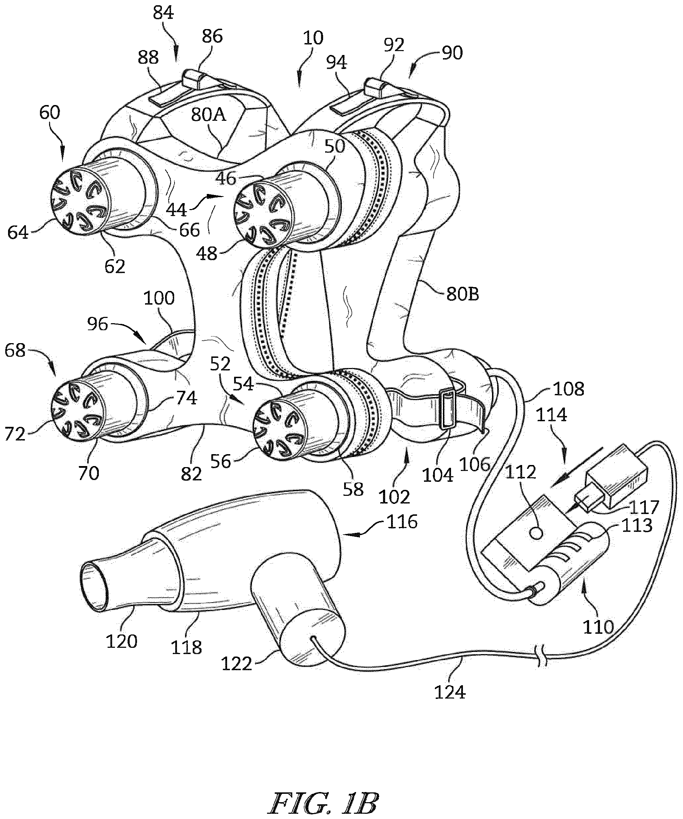

Controller 110 includes a user input 112, notches 113, and a female connection interface 115 as shown in Fig. 1A. Controller 110 sends signals to activate and control the operation of mechanical percussors 12, 20, 28, 36, 44, 52, 60, 68 of the apparatus 10. The controller 110, through user input 112, for example, controls the amount of force that the mechanical percussors 12, 20, 28, 36, 44, 52, 60, 68 generate to the patient's thorax. The user input 112 is shown diagrammatically in Figs. 1A and 1B as a single button but is intended to represent all types of user inputs including a plurality of buttons, a touch screen interface, and other user interfaces configured to receive input from a user. The illustrative example of the controller 110 has notches 113 that may include indicators therein to represent the battery level of a power supply 126, shown diagrammatically in Fig. 3, for instance.

In some embodiments, the patient has the option to adjust the force setting of the mechanical percussors 12, 20, 28, 36, 44, 52, 60, 68 to a desired level. In some embodiments, controller 110 may attach to clothing of the patient. For example, a belt clip, an alligator clip or other type of spring loaded clip, or any other means to attach controller 11 to a patient's clothing may be provided.

In the illustrative embodiment, the controller 110 connects to a nebulizer unit 116 through a connection interface 114. The illustrative nebulizer unit 116 includes a generally cylindrical housing 118 and a patient interface 120 extending axially from a first end of the housing 118. A nebulizer 122 extends generally radially from the housing 118 adjacent a second end of the housing 118. Nebulizer 122 serves as a handle for unit 116, if desired. Unit 116 includes a wire 124 that extends from the nebulizer 122 and that terminates at a male connection interface 117. Nebulizer 122 is operable to aerosolize and deliver drugs to the patient through internal passages provided in the housing 118 and the patient interface 120 in order to treat respiratory diseases or conditions of the patient using nebulizer unit 116.

In the illustrative example, the patient interface 120 is embodied as a mouthpiece. In other embodiments, the patient interface 120 may include, but is not limited to, a mask, a nasal cannula, and other means for delivering drugs to a patient through the use of a nebulizer 122, including one or more conduits or tubing extending from the housing 118. Combinations of tubing, conduits, a mouthpiece, a mask and a nasal cannula may be used as the patient interface 120, if desired. The patient interface 120, regardless of its configuration, allows for delivery of the aerosolized drugs created by the nebulizer 116 into the patient's airway. In some embodiments, the patient interface 120 is a disposable component that removably attaches to housing 118. Thus, after one or more uses, the patient discards the used patient interface 120 and replaces it with a new one.

In the illustrative embodiment, the connection interface 114 also includes a female connection interface 115 located on the controller 110. The male connection interface 117 of the nebulizer unit 116 at the terminal end of the wire 124 that extends from the nebulizer 122 couples with the female connection interface 115. Thus, interface 117 inserts into interface 115 to form an electrical connection between nebulizer 122 and circuitry of controller 110. In other embodiments, the connection interface 114 may include other arrangements. In the illustrative example, the controller 110 provides power from power supply 126, shown diagrammatically in Fig. 3, to the nebulizer 122. The controller 110 also controls and activates the nebulizer 122 through the user input 112. Thus, the patient turns the nebulizer 122 on and off using the user input 112 in some embodiments.

In some embodiments, the patient adjusts the operation of the nebulizer 122 using the user input 112. According to the present disclosure, the nebulizer 122 may be operated simultaneously with the operation of the mechanical percussors 12, 20, 28, 36, 44, 52, 60, 68, if desired. The nebulizer 122 may be turned off during operation of the mechanical percussors 12, 20, 28, 36, 44, 52, 60, 68 and the mechanical percussors 12, 20, 28, 36, 44, 52, 60, 68 may be turned off during the operation of the nebulizer 122 at the option of the user. Thus, apparatus has different modes of operation that are controlled by controller 110 based on inputs received at the user interface 112.

Nebulizer 122 is removably attached to housing 118 so that a liquid or powdered medicine may be added into the nebulizer 122. In some embodiments, nebulizer 122 comprises a vibrating mesh or vibrating plate which vibrates in response to an applied voltage or current to thereby aerosolize (aka nebulize or atomize) the liquid or powered medicine contained in the nebulizer. In alternative embodiments, nebulizer 122 operates pneumatically and a stream of pressurized air is communicated to the nebulizer 122 to aerosolize the medicine. In such alternative embodiments, controller 110 is enlarged sufficiently to house a pressure source, such as a pump, blower, compressor, or pressurized air reservoir that communicates pneumatically with nebulizer 122 through one or more conduits or tubes included in apparatus 10 in lieu of wire 124 and connector 117. However, controller 110 is able to be much more compact in size in embodiments of apparatus 10 having an electrically operated nebulizer 122.

Referring now to Fig. 2, a patient (in phantom) is shown wearing the apparatus 10. The nebulizer unit 116 is not shown in Fig. 2 to demonstrate that it may be detached from apparatus 10 and not used at all, if desired. In the illustrative embodiment, the positions of the mechanical percussors 12, 20, 28, 36, 44, 52, 60, 68 on the garment 11 are in the configuration as shown in Fig. 2. However, the mechanical percussors 12, 20, 28, 36, 44, 52, 60, 68 may be located elsewhere with respect to the patient in other embodiments. Different sizes of the garment 11 may be available for patients of varying body types, such a child, an average size adult, an obese adult, and so forth.

Referring now to Fig. 3, a block diagram of apparatus 10, including the electrical components contained in the controller 110 of the apparatus 10, is provided. The controller 110 includes power supply 126, a memory 128, and a microprocessor 130. In some embodiments, the power supply 126 is embodied as, but is not limited to, a rechargeable battery or batteries 126. The power supply 126 electrically couples to the female connection interface 115 of port 114 and also to memory 128 and microprocessor 130. The memory 128 connects with the microprocessor 130 and, in fact, these are shown in side-by-side fashion in Fig. 3 to indicate that they may be included in a single microcontroller in some embodiments.

The microprocessor 130 commands operation of the mechanical percussors 12, 20, 28, 36, 44, 52, 60, 68 and, in the illustrative example of Fig. 3, receives user inputs from a user interface 132, which, in some embodiments, also commands operation of nebulizer 122 via port 114. Thus, in response to user inputs at user input 112 and/or the user interface 132 the microprocessor 130 controls the mechanical percussors 12, 20, 28, 36, 44, 52, 60, 68 or the nebulizer 122 or both, as described above. In the illustrative embodiment, the connection interface 114 is a Universal Serial Bus (USB) port 114 between the controller 110 and the nebulizer 116. The controller 110 supplies power and, in some embodiments, controls the nebulizer 116 as described above. Alternatively or additionally, a user input similar to user input 112 is provided on nebulizer unit 116 to control operation of nebulizer 122.

The illustratively embodied garment 11 carries the mechanical percussors 12, 20, 28, 36, 44, 52, 60, 68 and also includes a storage component 134 as shown diagrammatically in Fig. 3. The storage component 134 may be illustratively embodied as, but is not limited to, a pocket 134 or strap (e.g., a stretchable or resilient strap). The storage component 134 is sized and configured to store the nebulizer unit 116 when not in use. In some embodiments, storage component 134 is a pocket of garment 11 that is provided for general use and not just storage of nebulizer unit 116. Alternatively or additionally, the storage component 134 may store spare patient interfaces 120 and/or controller 110.

Although certain illustrative embodiments have been described in detail above, variations and modifications exist.

## Claims

1. A high frequency chest wall oscillation apparatus (10) comprising,
a garment (11) to be worn by a patient,
a plurality of mechanical percussors (12, 20, 28, 36, 44, 52, 60, 68) carried by the garment,
a controller (110) spaced from the garment and hanging from the garment by a cable (108), and the controller signaling operation of the mechanical percussors,
an electrically operated nebulizer (116) having a connector (117) that removably plugs into a port (114) of the controller to receive power therefrom, and
a patient interface (120) to introduce nebulized particles from the electrically operated nebulizer into the patient's airway.

2. The apparatus (10) of claim 1, wherein the garment (11) comprises a vest having a front panel (80) and rear panel (82) coupled to the front panel by adjustable shoulder straps (84, 90) and adjustable side straps (96, 102).

3. The apparatus (10) of claim 2, wherein the front panel (80) comprises two separated parts (80A, 80B) connected by a full frontal closure (76).

4. The apparatus (10) of either claim 2 or claim 3, wherein a first set of the mechanical percussors (12, 20, 28, 36) of the plurality of mechanical percussors is carried by the front panel (80) and a second set of the mechanical percussors (44, 52, 60, 68) of the plurality of mechanical percussors is carried by the rear panel (82).

5. The apparatus (10) of claim 1, wherein the port (114) comprises a USB port.

6. The apparatus (10) of any preceding claim, wherein the controller (110) includes a power supply (126) electrically coupled to the electrically operated nebulizer (116).

7. The apparatus (10) of claim 6, wherein the power supply (126) comprises a rechargeable power supply.

8. The apparatus (10) of any preceding claim, wherein the controller (110) instructs the operation of the electrically operated nebulizer (116).

9. The apparatus (10) of any preceding claim, wherein the patient interface (120) to introduce nebulized particles from the electrically operated nebulizer (116) into the patient's airway comprises a mouthpiece or a mask.

10. The apparatus (10) of any preceding claim, wherein the controller (110) drives the electrically operated nebulizer (116).

11. The apparatus (10) of claim 10, wherein the controller (110) drives the electrically operated nebulizer (116) by electrical voltage.

12. The apparatus (10) of any preceding claim, wherein the electrically operated nebulizer (116) comprises a vibrating mesh nebulizer (122).

13. The apparatus (10) of any preceding claim, further comprising a switch to change between a plurality of different modes of operation.

14. The apparatus (10) of claim 13, wherein the modes of operation comprises
an on state and an off state for the plurality of mechanical percussors (12, 20, 28, 36, 44, 52, 60, 68),
an on state and an off state for the electrically operated nebulizer (116), and
an on state and an off state for the plurality of mechanical percussors and the electrically operated nebulizer.

## Patentansprüche

1. Eine Hochfrequenz-Brustwand-Vibrationsvorrichtung (10), umfassend
ein Kleidungsstück (11), das vom Patienten angezogen wird,
eine Vielzahl von mechanischen Perkussoren (12, 20, 28, 36, 44, 52, 60, 68), die vom Kleidungsstück getragen werden,
eine Steuereinheit (110), die sich in einem Abstand zum Kleidungsstück befindet und an einem Kabel (108) vom Kleidungsstück herunterhängt, und wobei die Steuereinheit den Betrieb der mechanischen Perkussoren anzeigt,
einen elektrisch betriebenen Nebulisator (116) mit einem Verbindungsstück (117), das entfernbar an einen Anschluss (114) der Steuereinheit anschließbar ist, um davon Strom aufzunehmen, und
eine Patientenschnittstelle (120), um vernebelte Partikel aus dem elektrisch betriebenen Nebulisator in die Atemwege des Patienten einzuleiten.

2. Die Vorrichtung (10) nach Anspruch 1, wobei das Kleidungsstück (11) eine Weste mit einer Frontplatte (80) und einer Rückenplatte (82) umfasst, die durch einstellbare Schultergurte (84, 90) und einstellbare Seitengurte (96, 102) an die Frontplatte gekoppelt ist.

3. Die Vorrichtung (10) nach Anspruch 2, wobei die Frontplatte (80) aus zwei separaten Teilen (80A, 80B) besteht, die durch einen Verschluss an der Vorderseite (76) vollständig verbunden werden können.

4. Die Vorrichtung (10) nach entweder Anspruch 2 oder Anspruch 3, wobei ein erster Satz der mechanischen Perkussoren (12, 20, 28, 36) der Vielzahl von mechanischen Perkussoren von der Frontplatte (80) getragen wird und ein zweiter Satz der mechanischen Perkussoren (44, 52, 60, 68) der Vielzahl von mechanischen Perkussoren von der Rückenplatte (82) getragen wird.

5. Die Vorrichtung (10) nach Anspruch 1, wobei der Anschluss (114) einen USB-Anschluss umfasst.

6. Die Vorrichtung (10) nach einem der vorstehenden Ansprüche, wobei die Steuereinheit (110) eine Stromversorgung (126) einschließt, die elektrisch an den elektrisch betriebenen Nebulisator (116) gekoppelt ist.

7. Die Vorrichtung (10) nach Anspruch 6, wobei die Stromversorgung (126) eine wiederaufladbare Stromversorgung umfasst.

8. Die Vorrichtung (10) nach einem der vorstehenden Ansprüche, wobei die Steuereinheit (110) Anweisungen zum Betrieb des elektrisch betriebenen Nebulisators (116) erteilt.

9. Die Vorrichtung (10) nach einem der vorstehenden Ansprüche, wobei die Patientenschnittstelle (120) ein Mundstück oder eine Maske umfasst, um vernebelte Partikel vom elektrisch betriebenen Nebulisator (116) in die Atemwege des Patienten einzuleiten.

10. Die Vorrichtung (10) nach einem der vorstehenden Ansprüche, wobei die Steuereinheit (110) den elektrisch betriebenen Nebulisator (116) antreibt.

11. Die Vorrichtung (10) nach Anspruch 10, wobei die Steuereinheit (110) den elektrisch betriebenen Nebulisator (116) durch elektrische Spannung antreibt.

12. Die Vorrichtung (10) nach einem der vorstehenden Ansprüche, wobei der elektrisch betriebene Nebulisator (116) einen vibrierenden Mesh-Nebulisator (122) umfasst.

13. Die Vorrichtung (10) nach einem der vorstehenden Ansprüche, weiter umfassend einen Schalter, um zwischen einer Vielzahl von verschiedenen Betriebsmodi zu wechseln.

14. Die Vorrichtung (10) nach Anspruch 13, wobei die Betriebsmodi
einen Ein-Zustand und einen Aus-Zustand für die Vielzahl von mechanischen Perkussoren (12, 20, 28, 36, 44, 52, 60, 68),
einen Ein-Zustand und einen Aus-Zustand für den elektrisch betriebenen Nebulisator (116), und
einen Ein-Zustand und einen Aus-Zustand für die Vielzahl von mechanischen Perkussoren und den elektrisch betriebenen Nebulisator umfassen.

## Revendications

1. Appareil d'oscillation de paroi thoracique haute fréquence (10) comprenant,
un vêtement (11) devant être porté par un patient,
une pluralité de percuteurs mécaniques (12, 20, 28, 36, 44, 52, 60, 68) positionnés sur le vêtement,
un dispositif de commande (110) à l'écart du vêtement et accroché au vêtement via un câble (108), et le dispositif de commande indiquant le fonctionnement des percuteurs mécaniques,
un nébuliseur à commande électrique (116) ayant un connecteur (117) qui se branche de manière amovible dans un port (114) du dispositif de commande afin de recevoir la puissance de celui-ci, et
une interface patient (120) pour introduire des particules nébulisées du nébuliseur à commande électrique dans les voies aériennes du patient.

2. Appareil (10) selon la revendication 1, dans lequel le vêtement (11) comprend un gilet ayant une face avant (80) et une face arrière (82) couplée à la face avant par des bretelles réglables (84, 90) et des sangles latérales réglables (96, 102).

3. Appareil (10) selon la revendication 2, dans lequel la face avant (80) comprend deux parties séparées (80A, 80B) reliées par une fermeture avant complète (76).

4. Appareil (10) selon la revendication 2 ou la revendication 3, dans lequel un premier ensemble de percuteurs mécaniques (12, 20, 28, 36) de la pluralité de percuteurs mécaniques est positionné sur la face avant (80) et un deuxième ensemble de percuteurs mécaniques (44, 52, 60, 68) de la pluralité de percuteurs mécaniques est positionné sur la face arrière (82).

5. Appareil (10) selon la revendication 1, dans lequel le port (114) comprend un port USB.

6. Appareil (10) selon l'une quelconque des revendications précédentes, dans lequel le dispositif de commande (110) inclut un bloc d'alimentation (126) couplé de manière électrique au nébuliseur à commande électrique (116).

7. Appareil (10) selon la revendication 6, dans lequel le bloc d'alimentation (126) comprend un bloc d'alimentation rechargeable.

8. Appareil (10) selon l'une quelconque des revendications précédentes, dans lequel le dispositif de commande (110) lance le fonctionnement du nébuliseur à commande électrique (116).

9. Appareil (10) selon l'une quelconque des revendications précédentes, dans lequel l'interface patient (120) pour introduire les particules nébulisées du nébuliseur à commande électrique (116) dans les voies aériennes du patient comprend un embout buccal ou un masque.

10. Appareil (10) selon l'une quelconque des revendications précédentes, dans lequel le dispositif de commande (110) entraîne le nébuliseur à commande électrique (116).

11. Appareil (10) selon la revendication 10, dans lequel le dispositif de commande (110) entraîne le nébuliseur à commande électrique (116) via la tension électrique.

12. Appareil (10) selon l'une quelconque des revendications précédentes, dans lequel le nébuliseur à commande électrique (116) comprend un nébuliseur à maille vibratoire (122).

13. Appareil (10) selon l'une quelconque des revendications précédentes, comprenant en outre un commutateur pour commuter entre une pluralité de modes de fonctionnement différents.

14. Appareil (10) selon la revendication 13, dans lequel les modes de fonctionnement comprennent
un état allumé et un état éteint pour la pluralité de percuteurs mécaniques (12, 20, 28, 36, 44, 52, 60, 68),
un état allumé et un état éteint pour le nébuliseur à commande électrique (116), et
un état allumé et un état éteint pour la pluralité de percuteurs mécaniques et le nébuliseur à commande électrique.
